# EUROPEAN PATENT SPECIFICATION

(11) **EP 4 493 210 B1**
(45) Date of publication and mention of the grant of the patent: **21.10.2026**
(21) Application number: 23712210.6
(22) Date of filing: 16.03.2023
(51) Int. Cl.: A61K 39/00, A61P 9/00, A61P 11/00, C07K 16/18, C07K 16/46

(54) **ANTI-ED-A ANTIBODIES FOR THE TREATMENT OF PULMONARY HYPERTENSION**
ANTI-ED-A-ANTIKÖRPER FÜR DIE BEHANDLUNG VON PULMONALER HYPERTENSION
ANTICORPS ANTI-ED-A POUR LE TRAITEMENT DE L'HYPERTENSION PULMONAIRE

(30) Priority: 17.03.2022 EP 22162841
(43) Date of publication of application: 22.01.2025
(73) Proprietor: Universitätsklinikum Jena, 07747 Jena (DE)
(72) Inventor: FRANZ, Marcus, 07747 Jena (DE); BERNDT, Alexander, 07747 Jena (DE); SCHULZE, Christian, 07747 Jena (DE)
(74) Representative: Mewburn Ellis LLP
(86) International application number: PCT/EP2023/056758
(87) International publication number: WO 2023/175077

(56) References cited:
- WO-A1-2022/018126
- FRANZ MARCUS ET AL: "Lung tissue remodelling in MCT-induced pulmonary hypertension: a proposal for a novel scoring system and changes in extracellular matrix and fibrosis associated gene expression", ONCOTARGET, vol. 7, no. 49, 8 November 2016 (2016-11-08), pages 81241 - 81254, XP055951980, DOI: 10.18632/oncotarget.13220
- BÄZ LAURA ET AL: "Serum Liberation of Fetal Fibronectin Variants in Patients with Pulmonary Hypertension: ED-A+ Fn as Promising Novel Biomarker of Pulmonary Vascular and Right Ventricular Myocardial Remodeling", JOURNAL OF CLINICAL MEDICINE, vol. 10, no. 12, 9 June 2021 (2021-06-09), pages 2559, XP055951982, DOI: 10.3390/jcm10122559
- ALI MD KHADEM ET AL: "Promising therapeutic approaches in pulmonary arterial hypertension", CURRENT OPINION IN PHARMACOLOGY, ELSEVIER SCIENCE PUBLISHERS, NL, vol. 59, 30 June 2021 (2021-06-30), pages 127 - 139, XP086718013, ISSN: 1471-4892, [retrieved on 20210630], DOI: 10.1016/J.COPH.2021.05.003

## Description

### Field of the Invention

The present application relates to the treatment of pulmonary hypertension (PH) using an antibody molecule, or antigen-binding fragment thereof, that binds the Extra Domain-A (ED-A) of fibronectin.

### Background

Pulmonary hypertension (PH) is a pathophysiological disorder that may involve multiple clinical conditions and is a consequence and comorbidity of the majority of cardiovascular and respiratory diseases. PH is a disease defined as an increase in mean pulmonary arterial pressure (PAPm) ≥25 mmHg at rest, as assessed by right heart catheterization (RHC). PH can be divided into five main groups: pulmonary arterial hypertension ("PAH") (Group 1), PH due to left heart disease ("LHD") (Group 2), PH due to lung diseases and/or hypoxaemia (Group 3), PH due to chronic pulmonary thrombembolisms (chronic thromboembolic PH = "CTEPH", Group 4), and PH with unclear and/or multifactorial mechanisms (Group 5) (Galiè N. et al. "2015 ESC/ERS Guidelines for the diagnosis and treatment of pulmonary hypertension - web addenda" (2016) European Heart Journal, Volume 37, Issue 1, pages 67-119). Current treatment options for PH in general act by decreasing vascular tone and thereby reducing pulmonary artery pressure. Most currently available treatment options are approved for PAH (Group 1), a rare disease, and are therefore not suitable for the treatment of the majority of patients suffering from other, more common, forms of PH (except for Group 4, in which the soluble guanylate cyclase (sGC) stimulator riociguat is approved) as mentioned above.

WO 2022/018126A1 relates to treatment of pulmonary hypertension using interleukin 9 (IL9), and in particular, IL9 conjugated to a binding member that binds to an antigen such as the ED-A of fibronectin.

The present invention has been devised in light of the above considerations.

### Summary of the Invention

The invention is defined by the claims and any other aspects, configurations, instances or embodiments set forth herein not falling within the scope of the claims are for information only. Any references in the description to methods of treatment (or diagnosis) refer to the compounds, pharmaceutical compositions and medicaments of the present invention for use in a method of treatment of the human (or animal) body by therapy (or for diagnosis).

The present inventors have shown that an anti-EDA antibody molecule improved clinical, hemodynamic and echocardiographic signs of PH in a mouse model of PH. Specifically, an attenuation of both the right ventricular systolic pressure, as well as surrogate markers of right ventricular load, as assessed by echocardiography, was observed. In contrast, administration of an antibody molecule to an irrelevant antigen did not attenuate disease severity in the same mouse model, showing that targeting of the ED-A of fibronectin was responsible for the observed effect.

The majority of currently approved treatments are for the treatment of PAH (Group 1), which is rare condition. These treatments generally act by decreasing vascular tone and thereby reducing pulmonary artery pressure. Common treatments for PAH include phosphodiesterase 5 inhibitors, endothelin receptor antagonists (such as Macitentan), soluble guanylate cyclase stimulators and prostanoids. PH due to left heart or lung disease (Group 2 and 3) are common secondary conditions resulting from a primary heart or lung condition, such as chronic left heart failure due to several etiologies, e.g., arterial hypertension or coronary artery disease, as well as chronic obstructive pulmonary disease, which is the most frequently occurring acquired lung disease, or pulmonary fibrosis. Here, therapy is usually focused on the treatment of the underlying disease. CTEPH (Group 4) is usually treated through the administration of anti-coagulants, if the obstruction is caused by blood clots. If anticoagulation does not improve the disease satisfactory, in particular when thrombotic material is organized or transformed to scar tissue, a pulmonary endarterectomy or balloon pulmonary angioplasty may be performed to improve blood flow and reduce pressure inside the arteries. In addition to or instead of surgery, which is an individualized decision depending on many patient characteristics, the soluble guanylate cyclase stimulator riociguat represents an approved pharmacological treatment option. Due to the diverse factors underlying the disease, there is no standardised treatment for PH with unclear and/or multifactorial mechanisms (Group 5).

The ED-A of fibronectin is known to be deposited in the extra-cellular matrix (ECM) during tissue remodelling and angiogenesis and expression of ED-A has been reported in lung tissue in spatial association to vessel structures and, to a lesser extent, in the lung parenchymal and stromal compartment, in a rat model of PH (Franz et al., Oncotarget, 2016, 7, 81241-81254). Moreover, the ED-A could also be shown to re-occur in remodeled right ventricular myocardium in animal models of PH (mouse model: Gouyou et al., Int. J. Mol. Sci., 2021, 22(7), 3460). In patients with PH of different clinical groups, relevant serum liberation of ED-A has been reported and proposed as a potential biomarker for initial diagnosis and aetiological differentiation, as well as, as a possible therapeutic target (Bäz et al., Int. J. Mol. Sci., 2020,21, 4174; Bäz et al., Journal of Clinical Medicine, 2021, 10, 2559). The ED-A of fibronectin, as well as other components of PH-associated tissue remodeling, was therefore thought to represent a possible target for the delivery of therapeutic agents to sites of disease in PH patients via immunoconjugates. Putative functional blocking of the molecule itself has also been mentioned but without any data or explanation to make credible any effect on PH resulting from such blocking, or details of how such blocking might be achieved (Bäz et al., Journal of Clinical Medicine, 2021, 10, 2559). The ability of an antibody, which binds ED-A itself, to improve PH disease severity was therefore completely unexpected and not predictable. In light of the data provided in the present application, targeting the ED-A of fibronectin in PH patients is expected to be suitable for the treatment of PH of different etiologies, in particular Group 1, Group 2 and Group 3 pulmonary hypertension, thereby representing a considerable improvement over currently available treatments.

The present invention thus relates to an antibody molecule which binds the ED-A of fibronectin for use in a method for treatment of pulmonary hypertension in a patient, as defined in the claims.

The pulmonary hypertension is Group 1, Group 2, or Group 3 pulmonary hypertension.

The antibody molecule may be an immunoglobulin G (IgG) molecule, in particular IgG1 or IgG4. The term "antibody molecule" encompasses an antigen-binding fragment thereof. Antigen-binding fragments of antibody molecules are known and include, for example, single-chain Fvs (scFvs), single-chain diabodies, and diabodies.

The antibody molecule binds to the ED-A of fibronectin. The antibody moleculecomprises an antigen-binding site having the complementarity determining regions (CDRs) of antibody F8 set forth in SEQ ID NOs 1 to 6. The antigen binding site comprises the VH and VL domains of antibody F8 set forth in SEQ ID NOs 7 and 8, respectively. In one preferred embodiment, the antibody molecule comprises or consists of the F8 IgG1 heavy and light chain amino acid sequences set forth in SEQ ID NOs: 13 and 14, respectively. In another preferred embodiment, the antibody molecule comprises or consists of the F8 IgG4 heavy and light chain amino acid sequences set forth in SEQ ID NOs: 17 and 14, respectively.

The antibody does not form part of a conjugate. That is, the antibody is not linked or otherwise conjugated to another moiety, such as interleukin-9 (IL9).

Treatment of PH using an antibody molecule which binds the ED-A of fibronectin has not been previously described.

The invention includes the combination of the aspects and preferred features described except where such a combination is clearly impermissible or expressly avoided.

### Summary of the Figures

Experiments illustrating the principles of the invention will now be discussed with reference to the accompanying figures in which:

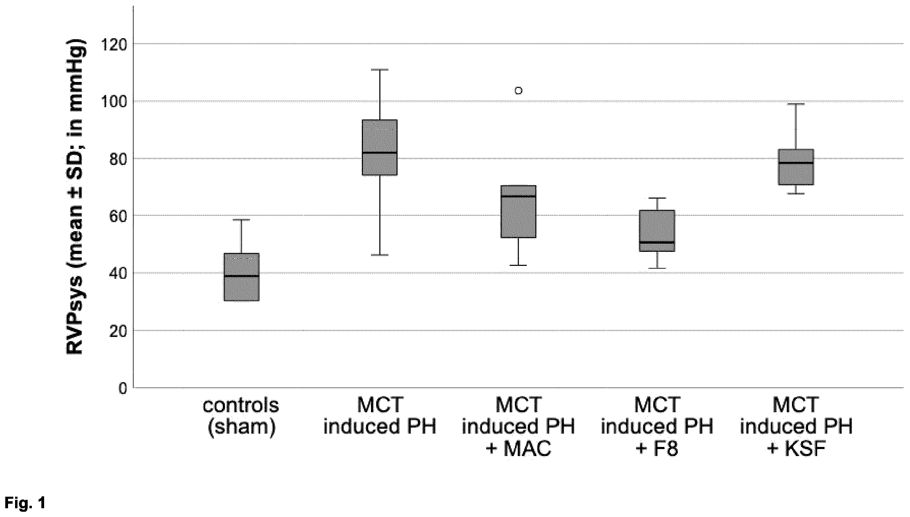

**Figure 1** shows systolic right ventricular pressure (RVPsys) values (mean ± standard deviation; in mmHg) in the five experimental groups. RVPsys was significantly elevated in all monocrotaline (MCT)-induced pulmonary hypertension (PH) groups (p<0.05) compared with the negative control, except for the group treated with F8 IgG (p=0.06). As compared to the MCT-induced PH group without treatment (positive control), a significant attenuation in RVPsys was only seen in the group treated with F8 IgG (p=0.010) and not in any of the other treatment groups including the group treated with macitentan (MAC) (p= not significant [n.s.]). In the group treated with F8 IgG, RVPsys was significantly reduced compared to the group treated with the negative control antibody KSF IgG (p=0.003).

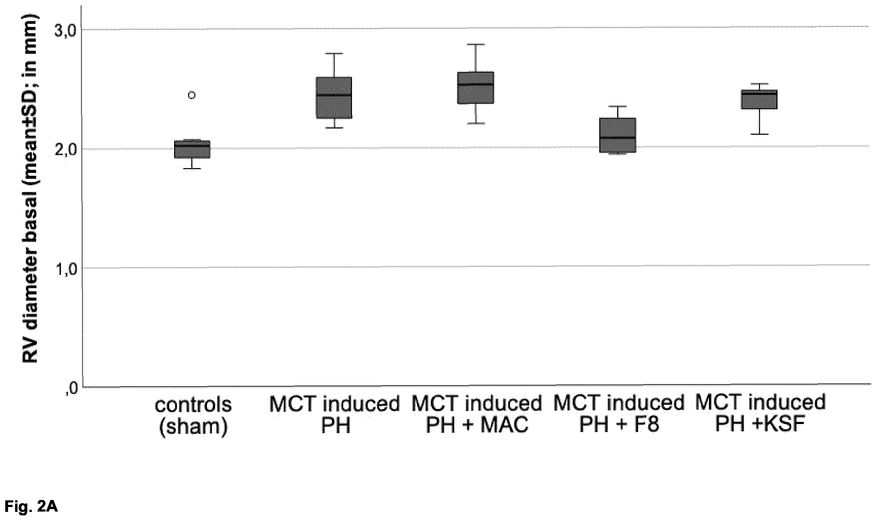

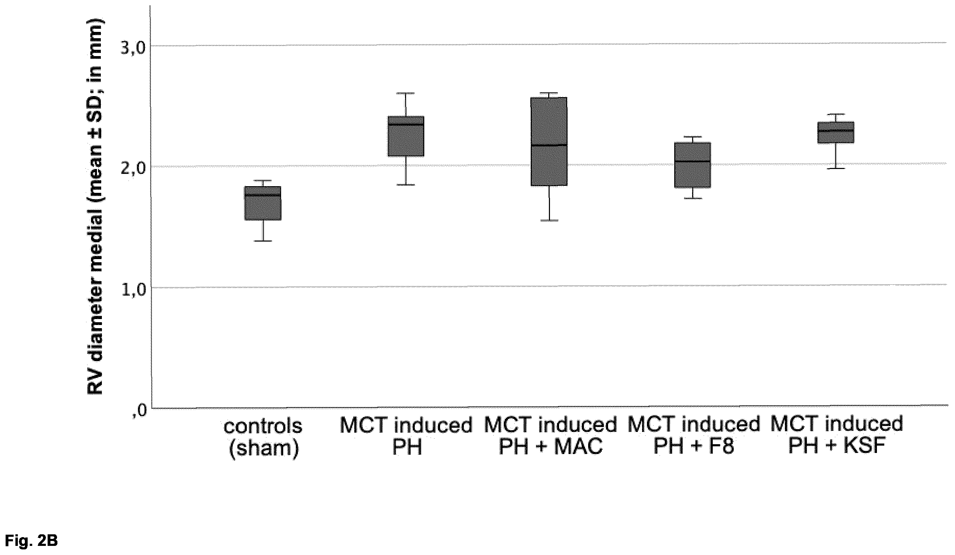

**Figures 2A** **and** **2B** show basal **(A)** and medial **(B)** right ventricular diameter (RV diameter) values (mean ± standard; in mm) in the five experimental groups. **(A):** RV basal diameter was significantly increased (p<0.05) in all MCT-induced PH groups compared with the negative controls, except for the group treated with F8 IgG (p=0.297). In the group treated with F8 IgG, a significant attenuation of RV basal diameter was observed compared with the MCT-induced PH group without treatment (p=0.002), the MCT-induced PH group with MAC treatment (p=0.005), and the MCT-induced PH group with KSF IgG treatment (p=0.005). **(B):** As compared to the negative controls, RV medial diameter was significantly increased in all MCT-induced PH groups (p<0.05), including the group treated with F8 IgG (p=0.028). In the group treated with F8 IgG, RV medial diameters were significantly reduced compared with the MCT-induced PH group without treatment (p=0.017) and the MCT-induced PH group with KSF IgG treatment (p=0.010).

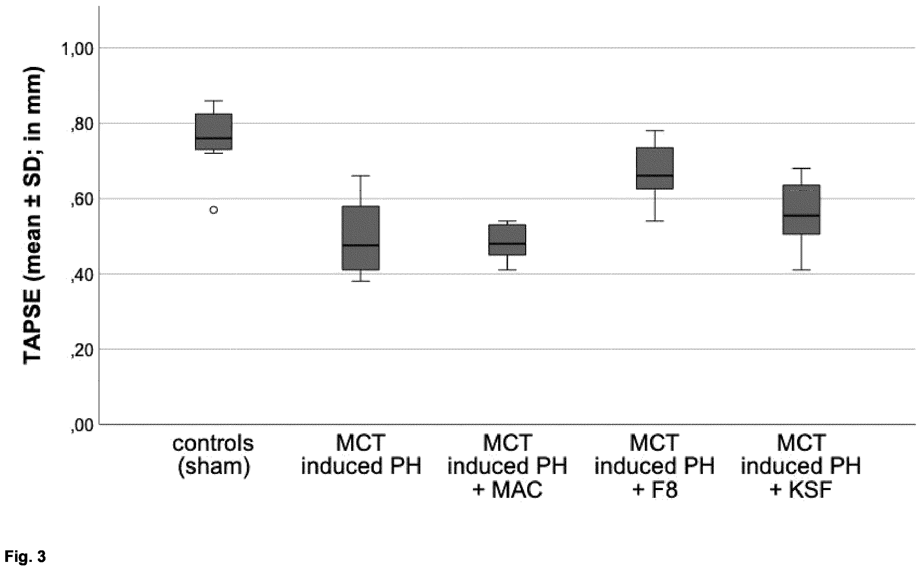

**Figure 3** shows tricuspid annular plane systolic excursion (TAPSE) values (mean ± standard; in mm) in the five experimental groups. As compared to negative controls, TAPSE was significantly reduced (p<0.05) in all MCT-induced PH groups except for the group treated with F8 IgG (p=0.110). A significant improvement in TAPSE was observed in the group treated with F8 IgG compared to the MCT-induced PH group without treatment (p=0.004), the MCT-induced PH group with MAC treatment (p=0.003), and the MCT-induced PH group with KSF IgG treatment (p=0.049).

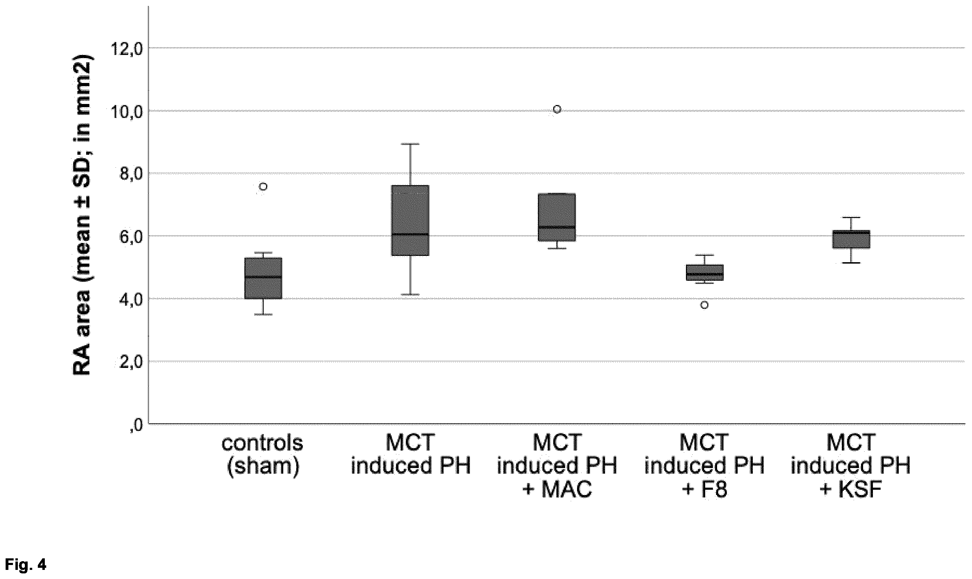

**Figure 4** shows right atrial area (RA area) values (mean ± standard; in mm) in the five experimental groups. As compared to negative controls, RA area was significantly increased (p<0.05) in all MCT-induced PH groups except for the group treated with F8 IgG (p=0.898). A significant reduction in RA area was observed in the group treated with F8 IgG as compared with the MCT-induced PH group without treatment (p=0.021), the MCT-induced PH group with MAC treatment (p=0.003), and the MCT-induced PH group with KSF IgG treatment (p=0.009).

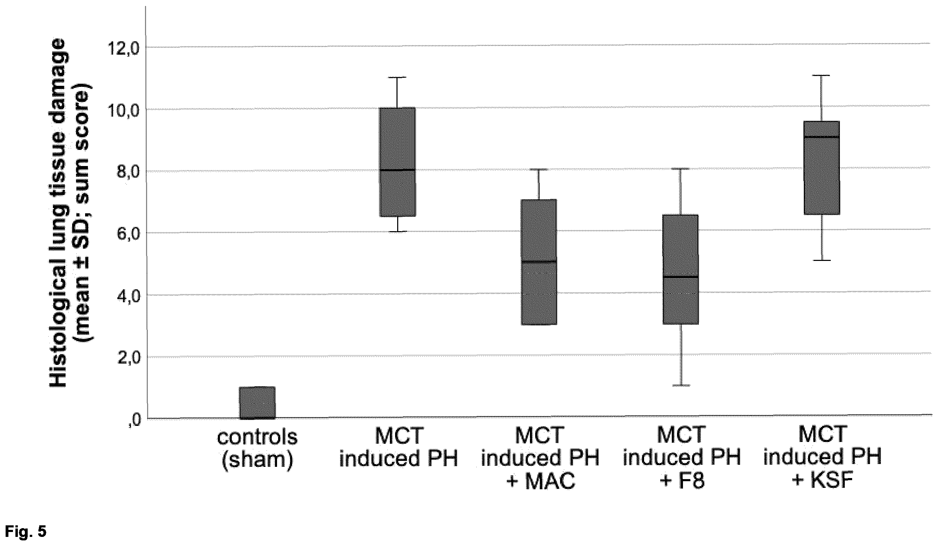

**Figure 5** shows microscopic lung tissue damage assessed by an established sum-score system (Franz et al., Oncotarget, 2016, 7(49):81241-81254) including all important histopathological parameters occurring in PH (mean ± standard; from 0 to 12 - arbitrary units) in the five experimental groups. As compared to negative controls, histopathological lung tissue damage was significantly increased (p<0.05) in all MCT-induced PH groups, including the group treated with F8 IgG (p=0.002). As compared to the MCT-induced PH group without treatment, lung tissue damage was significantly reduced in both the group treated with MAC (p=0.020) and the group treated with F8 IgG (p=0.005). No reduction in histopathological lung tissue damage was observed in the group treated with KSF IgG (p=0.907).

### Detailed Description of the Invention

Aspects of the present invention will now be discussed with reference to the accompanying figures. Further aspects will be apparent to those skilled in the art.

### ED-A Domain of Fibronectin

The alternatively spliced ED-A domain of fibronectin (ED-A) is a 90 amino acid sequence which is inserted into the extracellular matrix (ECM) component fibronectin (FN) through alternative splicing and is located between domain 11 and 12 of FN (Borsi et al. (1987), J. Cell. Biol.). The ED-As of mouse fibronectin and human fibronectin are 96.7% identical (only 3 amino acids differ between the two 90 amino acid sequences).

Expression of ED-A in the healthy adult is confined to vascular structures in few tissues in which physiological angiogenesis takes place, namely the placenta, the endometrium in the proliferative phase and some vessels in the ovaries (Schwager et al. (2009) Arthritis Res. Ther.). ED-A is also abundant during tissue remodeling, fibrosis (such as liver and pulmonary fibrosis), and in vascular tissue and stroma of many cancer types. Furthermore, the expression of ED-A in an MCT-induced model of pulmonary hypertension has been reported in Franz et al., Oncotarget, 2016, 7, 81241 - 81254. Current applicants have shown in WO2022/018126 that conjugates comprising IL9 and an anti-EDA binding member improved symptoms of PH in a mouse model of PH. Bäz et al. (J Clin Med (2021) 10, 2559) have reported that ED-A may be a promising novel biomarker of PH.

Over the years, the current applicant has developed a number of anti-cancer agents, including targeted cytokines ("immunocytokines") based on the anti-ED-A antibody "F8".

Reference to the work on the anti-ED-A "F8" antibody and conjugates thereof can be found in WO2008/120101, WO2009/013619, WO2009/056268, WO2010/078945, WO2010/078950, WO2011/015333, WO2012/041451, WO2013/014149, WO2014/055073, WO2014/173570, WO2014/174105, WO2015/114166, WO2016/180715, WO2017/009469, WO2018/069467, WO2018/087172, WO2018/224550, WO2019/185792, WO2020/070150, and WO2022/018126.

### Antibody molecules

The antibody molecules described herein may be whole antibody molecules or antigen binding fragments thereof. In some preferred embodiments, the antibody molecule comprises or consist of a single chain Fv (scFv), diabody, single-chain diabody, or an immunoglobulin (Ig) molecule, such as IgG. Most preferably, the antibody molecule is an IgG molecule, such as IgG1, IgG2, IgG3, or IgG4, preferably IgG1 or IgG4, most preferably IgG1.

An immunoglobulin molecule is composed of two light chains and two heavy chains that are disulfide-bonded. From the N- to C-terminus, each heavy chain comprises a variable region (VH), also called a variable heavy domain or a heavy chain variable domain, followed by three constant domains (CH1, CH2, and CH3), also called a heavy chain constant region. Similarly, from the N- to C-terminus, each light chain comprises a variable region (VL), also called a variable light domain or a light chain variable domain, followed by a light chain constant domain (CL), also called a light chain constant region. The heavy chain of an antibody molecule may be assigned to one of five types, called α (IgA), δ (IgD), ε (IgE), γ (IgG), or µ (IgM), some of which may be further divided into subtypes, e.g. γ1 (IgG1), γ2 (IgG2), y3 (IgG3), γ4 (IgG4), α1(IgA1) and α2 (IgA2). The light chain of an antibody molecule may be assigned to one of two types, called kappa (κ) and lambda (λ), based on the amino acid sequence of its constant domain. There are five major classes of immunoglobulins defined by the type of constant domain or constant region possessed by its heavy chain: IgA, IgD, IgE, IgG, and IgM, and several of these may be further divided into subclasses (isotypes), e.g., IgG1, IgG2, IgG3, IgG4, IgA1, and IgA2. The immunoglobulin heavy chain of an IgG molecule has the domain structure VH-CH1-CH2-CH3. The antibody light chain of an IgG antibody molecule has the domain structure VL-CL.

The antibody molecule preferably binds the Extra Domain-A (ED-A) of fibronectin.

Where the antibody molecule comprises more than one, e.g. two antibody molecules, the antibody molecules preferably have the same specificity (i.e. the antibody molecule is monospecific) and thus both bind to the ED-A of fibronectin.

The antibody molecule may comprise an antigen binding site having the complementarity determining regions (CDRs), or the VH and/or VL domains, of an antibody capable of binding to the ED-A of fibronectin. Antibodies which bind the ED-A of fibronectin are both known in the art and described herein. In addition, the provision of additional antibodies which bind the ED-A of fibronectin is well within the capabilities of the skilled person and could be employed in the treatment of pulmonary hypertension.

Thus, the antibody molecule may comprise an antigen binding site of antibody F8, which is known to bind the ED-A of fibronectin. The antibody molecule(s) may comprise an antigen binding site having one, two, three, four, five or six CDRs, or the VH and/or VL domains of antibody F8. The antibody molecule may comprise or consist of the sequence of antibody F8 in scFv format or, more preferably, in IgG format.

The antibody molecule may thus preferably comprise or consist of the sequence of the F8 antibody molecule in IgG format. For example, the F8 antibody molecule may be an IgG, IgA, IgE or IgM or any of the isotype sub-classes, particularly IgG1 or IgG4, with IgG1 being particularly preferred. IgG4 molecules exhibit reduced binding affinity to Fc receptors and reduced effector functions as compared to IgG1 molecules. In some embodiments, the IgG-class antibody molecule is an IgG1-subclass antibody molecule, particularly a human IgG1-subclass antibody molecule. In other embodiments, the IgG-class antibody molecule is an IgG4-subclass antibody molecule, particularly a human IgG4-subclass antibody molecule. In one embodiment, the IgG4-subclass antibody molecule comprises an amino acid substitution in the Fc region at position S228, specifically the amino acid substitution S228P numbered according to the EU numbering system (also called the EU index), corresponding to the amino acid substitution S226P in the F8 heavy chain amino acid sequence set forth in SEQ ID NO: 17. Optionally, the F8 heavy chain set forth in SEQ ID NO: 17 may further comprise a C-terminal lysine.

The amino acid sequences of the CDRs of F8 are:
SEQ ID NO:1 (CDR1 VH);
SEQ ID NO:2 (CDR2 VH);
SEQ ID NO:3 (CDR3 VH);
SEQ ID NO:4 (CDR1 VL);
SEQ ID NO:5 (CDR2 VL), and/or
SEQ ID NO:6 (CDR3 VL).

The amino acid sequences of the VH and VL of F8 are:
SEQ ID NO: 7 (VH)
SEQ ID NO: 8 (VL)

The amino acid sequences of the IgG1 heavy and light chains of F8 are:
SEQ ID NO: 13 (heavy chain)
SEQ ID NO: 14 (light chain)

The amino acid sequences of the IgG4 heavy and light chains of F8 are:
SEQ ID NO: 17 (heavy chain)
SEQ ID NO: 14 (light chain)

An antibody molecule may comprise a VH domain having at least 70%, more preferably one of at least 75%, 80%, 85%, 90%, 95%, 96%, 97%, 98%, 99% or 100%, sequence identity to the F8 VH domain amino acid sequence of SEQ ID NO: 7.

An antibody molecule may comprise a VL domain having at least 70%, more preferably one of at least 75%, 80%, 85%, 90%, 95%, 96%, 97%, 98%, 99% or 100%, sequence identity to the F8 VL domain amino acid sequence of SEQ ID NO: 8.

An antibody molecule may comprise a heavy chain having at least 70%, more preferably one of at least 75%, 80%, 85%, 90%, 95%, 96%, 97%, 98%, 99% or 100%, sequence identity to the F8 IgG1 heavy chain amino acid sequence of SEQ ID NO: 13.

An antibody molecule may comprise a heavy chain having at least 70%, more preferably one of at least 75%, 80%, 85%, 90%, 95%, 96%, 97%, 98%, 99% or 100%, sequence identity to the F8 IgG4 heavy chain amino acid sequence of SEQ ID NO: 17.

An antibody molecule may comprise a light chain having at least 70%, more preferably one of at least 75%, 80%, 85%, 90%, 95%, 96%, 97%, 98%, 99% or 100%, sequence identity to the F8 light chain amino acid sequence of SEQ ID NO: 14.

Sequence identity is commonly defined with reference to the algorithm GAP (Wisconsin GCG package, Accelerys Inc, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4. Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm (Altschul et al., Nucl. Acids Res. (1997) 25: 3389-3402) may be used.

Variants of the heavy and light chains, VH and VL domains, and CDRs may also be employed in an antibody molecule for use as described herein. Suitable variants can be obtained by means of methods of sequence alteration, or mutation, and screening. Particular variants for use as described herein may include one or more amino acid sequence alterations (addition, deletion, substitution and/or insertion of an amino acid residue), maybe less than about 20 alterations, less than about 15 alterations, less than about 10 alterations or less than about 5 alterations, 4, 3, 2 or 1. Alterations may be made in one or more framework regions and/or one or more CDRs. In particular, alterations may be made in VH CDR1, VH CDR2 and/or VH CDR3.

Preferably, the antibody molecule comprises the CDRs, VH and/or VL domains, or the heavy and light chain sequences of the F8 antibody in IgG format, in particular in IgG1 or IgG4 format.

### Pulmonary Hypertension

Pulmonary hypertension (PH) refers to high blood pressure in the blood vessels that supply blood to the lungs (pulmonary arteries). PH is defined as a mean pulmonary arterial pressure (PAPm) of ≥25 mmHg at rest, measured by right heart catheterization (RHC). The normal PAPm at rest in healthy individuals is 14 ± 3 mmHg with an upper limit of normal of approximately 20 mmHg (Galiè et al., ESC/ERS Guidelines for the diagnosis and treatment of pulmonary hypertension, European Respiratory Journal, 2015; 46: 903-975).

PH may be caused by heart or lung condition, associated with other medical conditions, such as connective tissue disorders or blood clots, or occur for unknown reasons. PH can be categorized into five groups according to their similar clinical presentation, pathological findings, haemodynamic characteristics and treatment strategy (Galiè et al., ESC/ERS Guidelines for the diagnosis and treatment of pulmonary hypertension, European Respiratory Journal, 2015; 46: 903-975):
Group 1: pulmonary arterial hypertension (PAH)
Group 2: PH due to left heart disease (LHD)
Group 3: PH due to lung disease and/or hypoxaemia
Group 4: Chronic thromboembolic pulmonary hypertension (CTEPH)
Group 5: PH with unclear and/or multifactorial mechanisms

The mouse model of PH employed by the present inventors is thought to mimic not only PAH (Group 1) but also other groups of PH, in particular group 2 and 3, insofar as they are in advanced stages and show pulmonary vascular remodelling as measured by elevated resistance in right heart catheterization (precapillary PH). Pulmonary vascular remodelling is the key structural alteration in PH and involves changes in intima, media, and adventitia of blood vessels, often with the interplay of inflammatory cells.

The pulmonary hypertension treated using an antibody molecule as described herein is preferably Group 1, Group 2, or Group 3 pulmonary hypertension, in particular as defined in Galiè et al., ESC/ERS Guidelines for the diagnosis and treatment of pulmonary hypertension, European Respiratory Journal, 2015; 46: 903-975.

### Methods of treatment

An antibody molecule as described herein may be used in a method of treatment of the human or animal body, such as a method of treatment (which may include prophylactic treatment and/or curative treatment) of a pulmonary hypertension in a patient (typically a human patient) comprising administering the antibody molecule to the patient.

Thus, an antibody molecule as herein described may be for use in a method of treating pulmonary hypertension, as defined by the claims.

The pulmonary hypertension treatable using an antibody molecule as described herein is Group 1, Group 2, or Group 3 pulmonary hypertension. Treatment may include prophylactic treatment.

### Pharmaceutical compositions

The antibody molecule may be in the form of a pharmaceutical composition comprising at least one antibody molecule and optionally a pharmaceutically acceptable excipient.

Pharmaceutical compositions typically comprise a therapeutically effective amount of an antibody molecule and optionally auxiliary substances such as pharmaceutically acceptable excipient(s). Said pharmaceutical compositions are prepared in a manner well known in the pharmaceutical art. A carrier or excipient may be a liquid material which can serve as a vehicle or medium for the active ingredient. Suitable carriers or excipients are well known in the art and include, for example, stabilisers, antioxidants, pH-regulating substances, controlled-release excipients. The pharmaceutical preparation of the invention may be adapted, for example, for parenteral use and may be administered to the patient in the form of solutions or the like.

Compositions comprising the antibody molecule may be administered to a patient. Administration is preferably in a "therapeutically effective amount", this being sufficient to show benefit to the patient. Such benefit may be at least amelioration of at least one symptom. The actual amount administered, and rate and time-course of administration, will depend on the nature and severity of what is being treated. Prescription of treatment, e.g. decisions on dosage etc., is within the responsibility of general practitioners and other medical doctors. Treatments may be repeated at daily, twice-weekly, weekly, or monthly intervals at the discretion of the physician.

Antibody molecules may be administered to a patient in need of treatment via any suitable route, usually by injection into the bloodstream and/or directly into the site to be treated. The precise dose and its frequency of administration will depend upon a number of factors, such as the route of treatment.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

A pharmaceutical composition comprising an antibody molecule described herein may be administered alone or in combination with other treatments, either simultaneously or sequentially dependent upon the type of pulmonary hypertension to be treated.

### Kits

Also described herein is a therapeutic kit for use in the treatment of pulmonary hypertension comprising an antibody molecule described herein. The components of a kit are preferably sterile and in sealed vials or other containers. A kit may further comprise instructions for use of the components in a method of the invention. The components of the kit may be comprised or packaged in a container, for example a bag, box, jar, tin or blister pack.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1: Activity of F8 IgG in a mouse model of monocrotaline (MCT)-induced pulmonary hypertension (PH)

### 1.1 Mouse model of MCT induced PH and treatment schedule

PH was induced in C57BL/6 mice (bodyweight: 25-30g). The animals were obtained from ZET facility (Zentale Experimentelle Tierhaltung) of the University Hospital Jena (UKJ, Jena, Germany). Prior to PH induction, mice were allowed to acclimatize for at least 7 days with ad libitum access to food and water as well as controlled light/dark cycles. 41 animals were investigated divided into the following five experimental groups:
1) sham induced controls (n=7)
2) MCT induced PH (n=12)
3) MCT induced PH + MAC (n=6)
4) MCT induced PH + F8 IgG (n=8)
5) MCT induced PH + KSF IgG (n=8)

For PH induction, the Monocrotaline (MCT, Carl Roth, Germany) method was used.

The F8 IgG and KSF IgG administered to the mice were chimeric antibodies, consisting of human VL and VH sequences fused to the murine CL and CH1-Hinge-CH2-CH3 sequences of the IgG subtype IgG2, respectively. These antibodies are also referred to as the F8-mlgG2a chimaera and KSF-mlgG2a chimaera herein. Murine IgG2a is known to functionally correspond to human IgG1.

Specifically, the amino acid sequences of the heavy and light chains of the F8 IgG antibody administered to the mice in the experiments were as follows:
SEQ ID NO: 11 (heavy chain)
SEQ ID NO: 12 (light chain)

The amino acid sequences of the heavy and light chains of the KSF IgG antibody administered to the mice in the experiments as follows:
SEQ ID NO: 15 (heavy chain)
SEQ ID NO: 16 (light chain)

The sham induced controls were injected with 30µl NaCl not containing MCT at day 1 (single dose; intraperitoneally, i.p.). These mice did not develop PH and thus served as healthy controls. The other 4 experimental groups were injected with MCT to induce PH (single dose; intraperitoneally, i.p.; 60 mg/kg body weight; volume 30 µl). Animals in the *MCT induced PH* + *MAC* group received the drug (Macitentan, Actelion Pharmaceuticals Ltd.) from day 14 to day 28 (once daily; per os; 15 mg/kg body weight). Animals in the MCT induced PH + F8 IgG group received F8 IgG 3 times on day 14, 16 and 18 (intravenously, i.v.; 195 µg/injection; volume 200 µl). Similarly, animals in the MCT induced PH + KSF IgG group received KSF IgG 3 times on day 14, 16 and 18 (intravenously, i.v.; 195 µg/injection; volume 120 µl).

To prevent infection and inflammatory alterations of the lungs, mice in all groups received Enrofloxacin 2.5 % ad water from day 1 to 14 after MCT injection.

All animals were weighed and examined twice weekly for clinical monitoring of well-being. The clinical condition was assessed using an established score (clinical severity score = CSS) from 1 to 5 (1 = no signs of clinical alterations, 2 = low-grade impairment, 3 = mid-grade impairment, 4 = high-grade impairment, 5 = exitus) obtained by evaluating spontaneous activity, reaction to exogenous stimuli and posture.

All experiments were conducted according to the National Institute of Health Guidelines for the Care and Use of Laboratory Animals (8th edition), to the European Community Council Directive for the Care and Use of Laboratory Animals of 22 September 2010 (2010/63/EU), the current version of the German Law on the Protection of Animals and the guidelines for animal care. The study protocol was approved by the appropriate State Office of Food Safety and Consumer Protection (TLLV, Bad Langensalza, Germany; local registration number: UKJ17-003).

### 1.2 Echocardiographic assessment

Echocardiographic assessment was performed on day 28 using the Vevo 770 Rodent-Ultrasound-System, Visual Sonic, Canada, 17MHz probe RMV176. Before echocardiography, mice were anesthetized with isoflurane for a duration time of less than 10 minutes (isoflurane-CP, 2.5V%, FiO2 1.0, oxygen per inhalation-flow dosage). Body temperature and respiratory rate were continuously monitored. All surrogate parameters of right ventricular (RV) morphology and function were assessed, among others, RV basal and medial diameters (in mm), RV length (in mm), tricuspid annular plane systolic excursion (TAPSE, in mm), right atrial area (RA area, in mm²) or main pulmonary artery diameter (MPA, in mm).

### 1.3 Right heart catheterization

On day 28 after MCT injection, mice of all experimental groups were deeply anesthetized with a single dose of 100 mg/kg body weight ketamine and 10 mg/kg body weight Xylazin in approximately 60 µl each administered i.p..

Right heart catheterization using a 1.4F micro conductance pressure-volume catheter (Model SPR-839; Millar Instruments Inc; PowerLab system, ADInstruments Ltd., Oxford, UK) was performed via the right vena jugularis interna to measure the systolic right ventricular pressure and thereby verify the success of the experimental setting. Mice were then sacrificed in deep anesthesia and analgesia to carry out cardiac blood collection after thoracotomy and to harvest the organs.

### 1.4 Histological evaluation

Microscopic evaluation to assess histopathological lung tissue damage was performed using H&E as well as Sirius Red stained lung tissue sections according to a defined sum-score (between 0 and 12; maximum score value = highest level of tissue damage) integrating certain histological alterations frequently occurring in PH: percentage of atelectasis area, percentage of emphysema area, degree of media hypertrophy of peribronchial arteries, presence of perivascular cellular edema of peribronchial arteries, and degree of media hypertrophy of small arteries.

### 1.5 Statistics

Statistical analyses were performed using IBM SPSS statistical software, version 28.0 (IBM SPSS Statistics for Windows. Armonk, NY, USA). Data are expressed as mean ± standard deviation. Kruskal-Wallis and Mann-Whitney-U test were used to test for significant differences between the different groups. A p-value < 0.05 as statistically significant.

### 1.6 Results

The main findings of the hemodynamic, echocardiographic and histopathological evaluation of this treatment study are presented in Figures 1 to 5.

PH can be successfully induced in C57BL/6 mice using the MCT method. Mice exhibit both significantly elevated right ventricular systolic pressure (RVPsys) values and distinct signs of right ventricular pressure overload with different degrees of right heart failure as assessed by echocardiography (including diameters of the right ventricle or tricuspid annular plane systolic excursion [TAPSE]). Moreover, mice exhibit relevant lung tissue damage in terms of typical PH-associated changes, e.g. media hypertrophy of peri-bronchial and small arteries, as detectable by microscopic analysis using a histopathological scoring system. All of these changes similarly occur in human patients with PH and have been proven to be of high clinical impact and prognostic relevance. Thus, the preclinical model used here is suitable for evaluating the effect of novel drugs in the treatment of PH, including the F8 antibody in IgG format, which specifically recognizes and functionally inhibits the extra-domain A (ED-A) of human fibronectin. ED-A is virtually absent in healthy human adult organs but becomes expressed during cardiovascular tissue remodelling processes, including PH and associated right heart failure. Without wishing to be bound by theory, the functional relevance of the ED-A of fibronectin in PH is thought to be attributable to the regulation of vascular smooth muscle cell (VSMC) activation and proliferation in the pulmonary vasculature, as well as the induction of fibroblast to myofibroblast (MyoFb) trans-differentiation in right ventricular myocardium. It is thought that by functional blocking of ED-A, these detrimental processes are attenuated, representing a novel disease modifying approach to stop or even reverse the disease.

In this example, the potential beneficial effects of the human recombinant F8 antibody specific for the ED-A domain of fibronectin in the full-length IgG format in the treatment of PH was tested and compared to treatment with the dual endothelin receptor antagonist (dual ERA) Macitentan (MAC), which represents an established therapy in a subgroup of human PH (group 1 PH = PAH according to current guidelines), and to treatment with IgG (KSF), which is specific for hen egg lysozyme, an irrelevant antigen, and thus acts as a negative control.

In contrast to treatment with KSF IgG, treatment of mice with F8 IgG was accompanied by:
1) a significant reduction of pressure values in the right ventricle (RVPsys, in mmHg) as the main pathophysiological surrogate of PH;
2) an improvement of a variety of echocardiographic signs of right ventricular load and dysfunction; and
3) an attenuation of lung tissue damage even at the histopathological level.

### 1.7 Conclusion

Taken together, in our preclinical PH model, the beneficial effects with respect to the majority of the parameters mentioned above, were superior to MAC treatment speaking well for the complex disease modifying action of F8 IgG in contrast to MAC, which primarily acts by pulmonary vasodilatation.

### Sequence Listing

Amino acid sequences of the F8 CDRs
   F8 CDR1 VH - LFT (SEQ ID NO: 1)
   F8 CDR2 VH - SGSGGS (SEQ ID NO: 2)
   F8 CDR3 VH - STHLYL (SEQ ID NO: 3)
   F8 CDR1 VL - MPF (SEQ ID NO: 4)
   F8 CDR2 VL - GASSRAT (SEQ ID NO: 5)
   F8 CDR3 VL - MRGRPP (SEQ ID NO: 6)
Amino acid sequence of the F8 VH domain (SEQ ID NO: 7) 
EVQLLESGGGLVQPGGSLRLSCAASGFTFSLFTMSWVRQAPGKGLEWVSAISGSGGSTYY
ADSVKGRFTISRDNSKNTLYLQMNSLRAEDTAVYYCAKSTHLYLFDYWGQGTLVTVSS
Amino acid sequence of the F8 VL domain (SEQ ID NO: 8) 
EIVLTQSPGTLSLSPGERATLSCRASQSVSMPFLAWYQQKPGQAPRLLIYGASSRATGIPD
RFSGSGSGTDFTLTISRLEPEDFAVYYCQQMRGRPPTFGQGTKVEIK
Amino acid sequence of the KSF VH domain (SEQ ID NO: 9) 
EVQLLESGGGLVQPGGSLRLSCAASGFTFSSYAMSWVRQAPGKGLEWVSAISGSGGSTY
YADSVKGRFTISRDNSKNTLYLQMNSLRAEDTAVYYCAKSPKVSLFDYWGQGTLVTVSS
Amino acid sequence of the KSF VL domain (SEQ ID NO: 10) 
SSELTQDPAVSVALGQTVRITCQGDSLRSYYASWYQQKPGQAPVLVIYGKNNRPSGIPDRF
SGSSSGNTASLTITGAQAEDEADYYCNSSPLNRLAVVFGGGTKLTVLG
Amino acid sequence of the F8-mlaG2a chimaera heavy chain (SEQ ID NO: 11)
   F8-VH (human variable heavy); murine CH1 (IgG2a); murine hinge (IgG2a);murine CH2 (IgG2a); murine CH3 (IgG2a) 
EVQLLESGGGLVQPGGSLRLSCAASGFTFSLFTMSWVRQAPGKGLEWVSAISGSGGSTYY
ADSVKGRFTISRDNSKNTLYLQMNSLRAEDTAVYYCAKSTHLYLFDYWGQGTLVTVSSAKT
TAPSVYPLAPVCGDTTGSSVTLGCLVKGYFPEPVTLTWNSGSLSSGVHTFPAVLQSDLYTL
SSSVTVTSSTWPSQSITCNVAHPASSTKVDKKIEPRGPTIKPCPPCKCPAPNLLGGPSVFIFP
PKIKDVLMISLSPIVTCVVVDVSEDDPDVQISWFVNNVEVHTAQTQTHREDYNSTLRVVSAL
PIQHQDWMSGKEFKCKVNNKDLPAPIERTISKPKGSVRAPQVYVLPPPEEEMTKKQVTLTC
MVTDFMPEDIYVEWTNNGKTELNYKNTEPVLDSDGSYFMYSKLRVEKKNWVERNSYSCSV
VHEGLHNHHTTKSFSRTPGK
Amino acid sequence of the F8-mlaG2a chimaera light chain (SEQ ID NO: 12)
   F8-VL (human variable light); murine CLk (murine constant light kappa) 
EIVLTQSPGTLSLSPGERATLSCRASQSVSMPFLAWYQQKPGQAPRLLIYGASSRATGIPD
RFSGSGSGTDFTLTISRLEPEDFAVYYCQQMRGRPPTFGQGTKVEIKRTDAAPTVSIFPPSS
EQLTSGGASVVCFLNNFYPKDINVKWKIDGSERQNGVLNSWTDQDSKDSTYSMSSTLTLT
KDEYERHNSYTCEATHKTSTSPIVKSFNRNEC
Amino acid sequence of the F8-IgG1 heavy chain (SEQ ID NO: 13)
   F8-VH (human variable heavy); human CH1 ((IgG1); human hinge (IgG1); human CH2 (IgG1); human CH3 (IgG1) 
EVQLLESGGGLVQPGGSLRLSCAASGFTFSLFTMSWVRQAPGKGLEWVSAISGSGGSTYY
ADSVKGRFTISRDNSKNTLYLQMNSLRAEDTAVYYCAKSTHLYLFDYWGQGTLVTVSSAST
KGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSC
SVMHEALHNHYTQKSLSLSPGK
Amino acid sequence of the F8-IgG1 and F8-IgG4 light chain (SEQ ID NO: 14)
   F8-VL (human variable light); human CLk (human constant light kappa) 
EIVLTQSPGTLSLSPGERATLSCRASQSVSMPFLAWYQQKPGQAPRLLIYGASSRATGIPD
RFSGSGSGTDFTLTISRLEPEDFAVYYCQQMRGRPPTFGQGTKVEIKRTVAAPSVFIFPPSD
EQLKSGTASVVCLLNNFYPREAKVQWKVDNALQSGNSQESVTEQDSKDSTYSLSSTLTLS
KADYEKHKVYACEVTHQGLSSPVTKSFNRGEC
Amino acid sequence of the KSF-mlgG2a chimaera heavy chain (SEQ ID NO: 15)
   KSF-VH (human variable heavy); murine CH1 (IgG2a); murine hinge (IgG2a); murine CH2 (IgG2a); murine CH3 (IgG2a) 
EVQLLESGGGLVQPGGSLRLSCAASGFTFSSYAMSWVRQAPGKGLEWVSAISGSGGSTY
YADSVKGRFTISRDNSKNTLYLQMNSLRAEDTAVYYCAKSPKVSLFDYWGQGTLVTVSSAK
TTAPSVYPLAPVCGDTTGSSVTLGCLVKGYFPEPVTLTWNSGSLSSGVHTFPAVLQSDLYT
LSSSVTVTSSTWPSQSITCNVAHPASSTKVDKKIEPRGPTIKPCPPCKCPAPNLLGGPSVFIF
PPKIKDVLMISLSPIVTCVVVDVSEDDPDVQISWFVNNVEVHTAQTQTHREDYNSTLRVVSA
LPIQHQDWMSGKEFKCKVNNKDLPAPIERTISKPKGSVRAPQVYVLPPPEEEMTKKQVTLT
 
CMVTDFMPEDIYVEWTNNGKTELNYKNTEPVLDSDGSYFMYSKLRVEKKNWVERNSYSCS
VVHEGLHNHHTTKSFSRTPGK
Amino acid sequence of the KSF-mlaG2a chimaera light chain (SEQ ID NO: 16)
   KSF-VL (human variable light); murine CLI (murine constant light lambda) 
SSELTQDPAVSVALGQTVRITCQGDSLRSYYASWYQQKPGQAPVLVIYGKNNRPSGIPDRF
SGSSSGNTASLTITGAQAEDEADYYCNSSPLNRLAVVFGGGTKLTVLGQPKSSPSVTLFPP
SSEELETNKATLVCTITDFYPGVVTVDWKVDGTPVTQGMETTQPSKQSNNKYMASSYLTLT
ARAWERHSSYSCQVTHEGHTVEKSLSRADCS
Amino acid sequence of the F8-IgG4 heavy chain (SEQ ID NO: 17)
   F8-VH (variable heavy); CH1-hIgG4; Hinge-hIgG4; **P (S226P);** CH2-hIgG4; CH3-hIgG4

EVQLLESGGGLVQPGGSLRLSCAASGFTFSLFTMSWVRQAPGKGLEWVSAISGSGGSTYY
ADSVKGRFTISRDNSKNTLYLQMNSLRAEDTAVYYCAKSTHLYLFDYWGQGTLVTVSSAST
KGPSVFPLAPCSRSTSESTAALGCLVKDYFPEPVTVSWNSGALTSGVHTFPAVLQSSGLYS
LSSVVTVPSSSLGTKTYTCNVDHKPSNTKVDKRVESKYGPPCPPCPAPEFLGGPSVFLFPP
KPKDTLMISRTPEVTCVVVDVSQEDPEVQFNWYVDGVEVHNAKTKPREEQFNSTYRVVSV
LTVLHQDWLNGKEYKCKVSNKGLPSSIEKTISKAKGQPREPQVYTLPPSQEEMTKNQVSLT
CLVKGFYPSDIAVEWESNGQPENNYKTTPPVLDSDGSFFLYSRLTVDKSRWQEGNVFSCS
VMHEALHNHYTQKSLSLSLG

## Claims

1. An antibody molecule, which binds the Extra Domain-A (EDA) of fibronectin, for use in a method for treatment of Group 1, Group 2, or Group 3 pulmonary hypertension in a patient, wherein the antibody molecule does not form part of a conjugate, wherein the antibody molecule comprises a VH domain comprising a set of complementarity determining regions HCDR1, HCDR2 and HCDR3, and a VL domain comprising a set of complementarity determining regions LCDR1, LCDR2 and LCDR3, and wherein:
the HCDR1, HCDR2 and HCDR3 comprise the amino acid sequences set forth in SEQ ID NOs 1, 2 and 3, respectively;
the LCDR1, LCDR2 and LCDR3 comprise the amino acid sequences set forth in SEQ ID NOs 4, 5 and 6, respectively; and
the antibody molecule comprises the VH and VL domains set forth in SEQ ID NOs 7 and 8, respectively.

2. The antibody molecule for use according to claim 1, wherein the antibody molecule is an immunoglobulin G (IgG) molecule.

3. The antibody molecule for use according to claim 2, wherein the antibody molecule is an IgG1 or IgG4 molecule.

4. The antibody molecule for use according to any one of claims 1 to 3, wherein the antibody molecule comprises or consists of the IgG1 heavy chain set forth in SEQ ID NO: 13, and the light chain set forth in SEQ ID NO: 14.

5. The antibody molecule for use according to any one of claims 1 to 3, wherein the antibody molecule comprises or consists of the IgG4 heavy chain set forth in SEQ ID NO: 17, and the light chain set forth in SEQ ID NO: 14.

## Patentansprüche

1. Antikörpermolekül, das die Extra-Domäne A (EDA) von Fibronektin bindet, zur Verwendung in einem Verfahren zur Behandlung von pulmonaler Hypertonie der Gruppe 1, Gruppe 2 oder Gruppe 3 bei einem Patienten, wobei das Antikörpermolekül nicht Teil eines Konjugats ist, wobei das Antikörpermolekül eine VH-Domäne, die einen Satz von komplementaritätsbestimmenden Regionen HCDR1, HCDR2 und HCDR3 umfasst, und eine VL-Domäne, die einen Satz von komplementaritätsbestimmenden Regionen LCDR1, LCDR2 und LCDR3 umfasst, umfasst und wobei:
die HCDR1, HCDR2 und CHDR3 die in SEQ ID NO: 1, 2 bzw. 3 angegebene Aminosäuresequenz umfassen;
die LCDR1, LCDR2 und LCDR3 die in SEQ ID NO: 4, 5 bzw. 6 angegebene Aminosäuresequenz umfassen; und
das Antikörpermolekül die in SEQ ID NO: 7 bzw. 8 angegebene VH- und VL-Domäne umfasst.

2. Antikörpermolekül zur Verwendung nach Anspruch 1, wobei das Antikörpermolekül ein Immunglobulin-G- (IgG-) Molekül ist.

3. Antikörpermolekül zur Verwendung nach Anspruch 2, wobei das Antikörpermolekül ein IgG1- oder IgG4-Molekül ist.

4. Antikörpermolekül zur Verwendung nach einem der Ansprüche 1 bis 3, wobei das Antikörpermolekül die in SEQ ID NO: 13 angegebene schwere Kette von IgG1 und die in SEQ ID NO: 14 angegebene leichte Kette umfasst oder daraus besteht.

5. Antikörpermolekül zur Verwendung nach einem der Ansprüche 1 bis 3, wobei das Antikörpermolekül die in SEQ ID NO: 17 angegebene schwere Kette von IgG4 und die in SEQ ID NO: 14 angegebene leichte Kette umfasst oder daraus besteht.

## Revendications

1. Molécule d'anticorps, qui se lie au domaine supplémentaire-A (ED-A) de la fibronectine, pour utilisation dans un procédé de traitement de l'hypertension pulmonaire de groupe 1, de groupe 2 ou de groupe 3 chez un patient, dans laquelle la molécule d'anticorps ne fait pas partie d'un conjugué, dans laquelle la molécule d'anticorps comprend un domaine VH comprenant un ensemble de régions de détermination de complémentarité HCDR1, HCDR2 et HCDR3, et un domaine VL comprenant un ensemble de régions de détermination de complémentarité LCDR1, LCDR2 et LCDR3, et dans laquelle :
les HCDR1, HCDR2 et HCDR3 comprennent les séquences d'acides aminés énoncées respectivement dans SEQ ID NOs 1, 2 et 3 ;
les LCDR1, LCDR2 et LCDR3 comprennent les séquences d'acides aminés énoncées respectivement dans SEQ ID NOs 4, 5 et 6 ; et
la molécule d'anticorps comprend les domaines VH et VL énoncés dans respectivement, SEQ ID N° 7 et 8.

2. Molécule d'anticorps pour utilisation selon la revendication 1, dans laquelle la molécule d'anticorps est une molécule d'immunoglobuline G (IgG).

3. Molécule d'anticorps pour utilisation selon la revendication 2, dans laquelle la molécule d'anticorps est une molécule d'IgG1 ou d'IgG4.

4. Molécule d'anticorps pour utilisation selon l'une quelconque des revendications 1 à 3, dans laquelle la molécule d'anticorps comprend ou consiste en la chaîne lourde d'IgG1 énoncée dans SEQ ID NO : 13, et la chaîne légère énoncée dans SEQ ID NO : 14.

5. Molécule d'anticorps pour utilisation selon l'une quelconque des revendications 1 à 3, dans laquelle la molécule d'anticorps comprend ou consiste en la chaîne lourde d'IgG4 énoncée dans SEQ ID NO : 17, et la chaîne légère énoncée dans SEQ ID NO : 14.
